(19) Europäisches Patentamt
European Patent Office
Office européen des brevets

(11) Publication number: **0 295 269 B1**

(12) # EUROPEAN PATENT SPECIFICATION

(45) Date of publication of the patent specification: **21.03.90**

(51) Int. Cl.[5]: **B 01 J 35/00, B 01 J 21/00**

(21) Application number: **87904346.1**

(22) Date of filing: **11.06.87**

(86) International application number: **PCT/US 87/01445**

(87) International publication number: **WO 88/00093 (14.01.88 Gazette 88/02)**

(54) **LAYERED METAL OXIDES CONTAINING INTERLAYER OXIDES AND THEIR SYNTHESIS.**

(30) Priority: **27.06.86 US 879787**

(43) Date of publication of application: **21.12.88 Bulletin 88/51**

(45) Publication of the grant of the patent: **21.03.90 Bulletin 90/12**

(84) Designated Contracting States: **BE DE FR GB IT NL SE**

(56) References cited:
EP-A-0 131 685
EP-A-0 205 711
US-A-4 151 123
US-A-4 637 991
US-A-4 637 992

(73) Proprietor: **MOBIL OIL CORPORATION**
**150 East 42nd Street**
**New York New York 10017 (US)**

(72) Inventor: **AUFDEMBRINK, Brent, Allen**
**5 Christopher Road**
**Voorhees, NJ 08043 (US)**

(74) Representative: **Colmer, Stephen Gary**
**Patent Department c/o Mobil Services Company Limited**
**Mobil Court 3 Clements Inn**
**London WC2A 2EB (GB)**

**EP 0 295 269 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBERGRAF, STOCKHOLM 1990

## Description

The present invention relates to layered metal oxides containing interlayer polymeric oxides as well as a method for preparing the same.

Many layered materials are known which have three-dimensional structures which exhibit their strongest chemical bonding in only two dimensions. In such materials, the stronger chemical bonds are formed in two-dimensional planes and a three-dimensional solid is formed by stacking such planes on top of each other, however, the interactions between the planes being weaker than the chemical bonds holding an individual plane together. The weaker bonds generally arise from interlayer attractions such as Van der Waals forces, electrostatic interactions, and hydrogen bonding. In those situations where the layered structure has electronically neutral sheets interacting with each other solely through Van der Waals forces, a high degree of lubricity is manifested as the planes slide across each other without encountering the energy barriers that arise with strong interlayer bonding. Graphite is an example of such a material. The silicate layers of a number of clay materials are held together by electrostatic attraction provided by ions located between the layers. In addition, hydrogen bonding interactions can occur directly between complementary sites on adjacent layers, or can be provided by interlamellar bridging molecules.

Laminated materials such as clays may be modified to increase their surface area. In particular, the distance between the layers can be increased substantially by absorption of various swelling agents such as water, ethylene glycol, amines and ketones, which enter the interlamellar space and push the layers apart. However, the interlamellar spaces of such layered materials tend to collapse when the molecules occupying the space are removed by, for example, exposing the clays to high temperatures. Accordingly, such layered materials having enhanced surface area are not suited for use in chemical processes involving even moderately severe conditions.

The extent of interlayer separation can be estimated by using standard techniques such as X-ray diffraction to determine the basal spacing, also known as "repeat distance" or "d-spacing". These values indicate the distance between, for example, the uppermost margin of one layer with the uppermost margin of its adjoining layer. If the layer thickness is known, the interlayer spacing can be determined by subtracting the layer thickness from the basal spacing.

Various approaches have been taken to provide layered materials of enhanced interlayer distance having thermal stability. Most techniques rely upon the introduction of an inorganic "pillaring" agent between the layers of a layered material. For example, U.S. Patent 4 216 188 discloses a clay which is cross-linked with metal hydroxide prepared from a highly dilute colloidal solution containing fully separated unit layers and a crosslinking agent comprising a colloidal metal hydroxide solution. However, this method requires a highly dilute forming solution of the clay (less than 1 g/l) in order to effect full layer separation prior to incorporation of the pillaring species, as well as positively charged species of cross linking agents.

U.S. Patent 4 248 739 describes stable pillared interlayered clay prepared from smectite clays reacted with cationic metal complexes of metals such as aluminum and zirconium. The resulting products exhibit high interlayer separation and thermal stability.

U.S. Patent 4 176 090 discloses a clay composition interlayered with polymeric cationic hydroxy metal complexes of metals such as aluminum, zirconium and titanium. Interlayer distances of up to 1.6 nm are claimed although only distances restricted to about 0.9 nm are exemplified for calcined samples. These distances are essentially unvariable and related to the specific size of the hydroxy metal complex.

Silicon-containing materials are believed to be a highly desirable species of pillaring agent owing to their high thermal stability characteristics. U.S. Patent 4 367 163 describes a clay intercalated with silica prepared by impregnating a clay substrate with a silicon-containing reactant such as an ionic silicon complex, e.g., silicon acetylacetonate, or a neutral species such as $SiCl_4$. The clay may be swelled prior to or during silicon impregnation with a suitable polar solvent such as methylene chloride, acetone, benzaldehyde, tri- or tetraalkylammonium ions, or dimethylsulfoxide. This method, however, appears to provide only a monolayer of intercalated silica resulting in a product of small spacing between layers, about 0.2 – 0.3 nm as determined by X-ray diffraction.

In one aspect, the present invention resides in a layered product comprising a layered oxide in which the layers have a perovskite-related structure and pillars of an oxide of at least one element selected from Groups IB, IIB, IIIA, IIIB, IVA, IVB, VA, VB, VIA, VIIA and VIIIA of the periodic Table of the Elements (Fisher Scientific Co. Cat. No. 5-702-10, 1978) separating the layers of the layered oxide.

Preferably, the pillars comprise a polymeric oxide of an element of Group IVB and most preferably polymeric silica.

In another aspect, the present invention relates to a method for preparing a layered product comprising a layered oxide in which the layers have a perovskite-related structure and are separated by pillars containing an oxide of at least one element selected from Groups IB, IIB, IIIA, IIIB, IVA, IVB, VA, VB, VIA, VIIA, and VIIIA of the Periodic Table which method comprises the steps of physically separating the layers of the layered oxide by introducing an organic cationic species between the layers at interlayer anionic sites associated with the layered oxide, introducing between the separated layers of the layered oxide a compound capable of conversion to an oxide and then converting said compound to the oxide to form oxide pillars separating adjacent layers of

the layered oxide.

It is to be understood that the term "layered" oxide is used herein in its commonly accepted sense to refer to a material which comprises a plurality of separate oxide layers which are capable of being physically displaced away from one another such that the spacing between adjacent layers is increased. Such displacement can be measured by X-ray diffraction techniques and/or by density measurements.

The present invention is particularly useful in that it permits the preparation of pillared oxide materials of relatively high interplanar distance (d-spacing), e.g., greater than about 1 nm and preferably greater than 2 nm up to and even exceeding 3 nm. These materials are capable of being exposed to severe conditions such as those encountered in calcining, e.g., at temperatures of about 450°C for about two or more hours, e.g., four hours, in nitrogen or air, without significant decrease, say, e.g., less than about 10 %, in interlayer distance. Furthermore, such pillared oxides can be prepared without the severe dilution often necessary to introduce the interspathic material in prior art techniques of interlayering. Finally, the size of interspathic oxide contained within the final product can be greatly varied because the oxide precursor species is introduced in an electrically neutral form such that the amount of interspathic material incorporated within the layered oxide is not dependent upon the charge density of the original layered oxide. Charge density should be taken into consideration in determining the suitability of the cationic species introduced between the layers in the procedure used to prop open the layers prior to pillaring.

The present invention utilizes a layered oxide starting material in which the layers have a perovskite type structure and contain anionic sites having interspathic cat ions associated therewith. Such interspathic cations may include hydrogen ion, hydronium ion and alkali metal cation.

Perovskite-related layered oxides are known in the art and are described, for example by Dion M, Ganne M., Tournoux M, in Mat. Res. Bull. 1981, 16, 1429; Galasso F., "Structure properties and Preparation of Perovskite Type Compounds", Pergamon Press, 1969 and Jacobson et al., Inorg. Chem., 1985, 24, 3727. These materials as well as their organic-swelled analogues, e.g., those which are octylamine-swelled, are disclosed in U.S. Patent No. 4 593 013.

The perovskite-related layered-oxides used herein may be represented by the formula

$M_m[A_{n-1}B_nO_{3n+1}]$,

although oxygen-deficient variants of this formula are known (see, for example Goodenough J.B., "Progress in Solid State Chemisty", Vol. 5., Pergamon Press, ed. H. Reiss, page 342) and may also be employed. In this formula M is a charge-balancing interspathic cation; $[A_{n-1}B_nO_{3m+1}]$ represents a perovskite-like layer wherein A is one or more metal atoms capable of occupying 12-coordinate sites and B is a metal atom capable of occupying 6-coordinate sites, m is greater than 0 and preferably is less than or equal to 1 and n is greater than or equal to 2 and preferably is 3 to 7. Each layer comprises a cubic arrangement of corner-shared $BO_6$ octahedra with A occupying a 12-coordinated site in the center of each cube. For purposes of the present invention, the term "cubic arrangement" can include any generally cubic or pseudo-cubic arrangement.

The thickness of each layer in terms of $BO_6$ octahedra is denoted by n. In other words, the layers can vary, for example, between 2 and 7 $BO_6$ octahedra in thickness, depending on the perovskite-like layered material. Preferably the layers have a low charge density in order to exhibit the ion exchange properties necessary for incorporation of the organic cationic propping agent prior to intercalation with the polymeric oxide precursor. Although some perovskite-like layered materials have a charge density per formula unit of two or more, the perovskite-like layered materials used in the present invention preferably have a charge density of one or less. However, it is possible that a propping agent of requisite shape and charge can exchanged with the interspathic cations in materials where m is greater than 1.

M can be a monovalent, divalent or trivalent cation, preferably a monovalent cation selected from Li, Na, K, Rb, Cs, $NH_4$ and H, while A can be one or more mono-, di- or trivalent metals selected from Groups IA, IIA, IIIB and the lanthanides and B can be one or more transition metals selected from Re and Groups IVB, VB and VIB. In one preferred embodiment, $A_{n-1}$ can be $Ca_2Na_{n-3}$ and B is Nb; in other words, each perovskite layer is represented by the formula

$Ca_2Na_{n-3}Nb_nO_{3n+1}$

Preferably in such cases, M is K and n is 3, so that the overal layered oxide starting material has the formula

$KCa_2Nb_3O_{10}$

In the method of the invention, the layered oxide starting material is treated with a "propping" agent comprising a source of organic cation such as organoammonium cation in order to effect an exchange of or addition to the interlayer cations in the starting material resulting in the layers of the starting material being propped apart. The source of organic cation in those instances where the interlayer cations include hydrogen or hydronium ions may include a neutral compound such as organic amine which is converted to a cationic analogue during the "propping" treatment. In some instances, it may be desirable to remove excess propping agent which is not electrostatically bound within the layered starting material in

order to permit the subsequent addition of greater amounts of polymeric oxide precursor. Such removal may be effected by washing out the propping agent with a suitable solvent.

The foregoing treatment can result in the formation of a layered metal oxide of enhanced interlayer separation depending upon the size of the organic cation introduced. In one embodiment, a series of organic cation exchanges is carried out. For example, an organic cation may be exchanged with an organic cation of greater size, thus increasing the interlayer separation in a step-wise fashion. Preferably contact of the layered oxide with the propping agent is conducted in aqueous medium and at a temperature above ambient, preferably 70 – 110°C.

After the ion exchange, the organic-"propped" species is treated with a compound capable of conversion, preferably by hydrolysis, to a polymeric oxide. Preferably, such treatment is conducted above ambient temperature, conveniently 70 – 100°C and most preferably 80 – 90°C. The "propped" layered material containing the polymeric oxide precursor is then treated to produce polymeric oxide pillars separating the substrate layers. Where the treatment involves hydrolysis, this may be carried out using the water already present in organic-"propped" layered oxide material. In this case, the extent of hydrolysis may be modified by varying the extent to which the organic-"propped" species is dried prior to addition of the polymeric oxide precursor.

It is preferred that the organic cation deposited between the layers be capable of being removed from the layered oxide material without substantial disturbance or removal of the interspathic polymeric oxide. For example, organic cations such as n-octylammonium may be removed by exposure to elevated temperatures, e.g., calcination, in nitrogen or air or chemical oxidation conditions, preferably after the interspathic polymeric oxide precursor has been converted to the polymeric oxide pillars in order to form the layered material of the present invention.

The products of the present invention, especially when calcined, exhibit high surface area, e.g., greater than 200, 300, 400 or even 600 $m^2/g$, and thermal and hydrothermal stability making them highly useful as catalysts or catalytic supports for hydrocarbon conversion processes, for example, cracking and hydrocracking.

According to the method of the invention, the layered perovskite-type starting material is subjected to a swelling or propping step in which the material is treated with an organic compound capable of forming cationic species such as organophosphonium or organoammonium ion, preferably at a temperature of 70 – 110°C, e.g. 100°C. Insertion of the organic cation between the adjoining layers serves to physically separate the layers in such a way as to make the layered perovskite-type material receptive to the interlayer addition of an electrically neutral, hydrolyzable, polymeric oxide precursor. In particular, alkylammonium cations have been found useful in the present invention. Thus $C_3$ and larger alkylammonium, e.g., n-octylammonium, cations are readily incorporated within the interlayer spaces of the layered perovskite-type material, serving to prop open the layers in such a way as to allow incorporation of the polymeric oxide precursor. The extent of the interlayer spacing can be controlled by the size of the organoammonium ion employed so that use of the n-propylammonium cation can achieve an interlayer opening of 0.2 to 0.5 nm whereas to achieve an interlayer opening of 1 to 2 nm an n-octylammonium cation or a cation of equivalent length is required. Indeed, the size and shape of the organic cation can affect whether or not it can be incorporated within the layered structure at all. For example, bulky cations such as tetrapropylammonium are generally undesirable for use in the present method while n-alkyl ammonium cations such as those derived from n-alkyl primary amines and $R_3R'N^+$ cations whre R is methyl or ethyl and R' is an alkyl group with at least 5 carbon atoms are preferred. The organic ammonium cations separating the perovskite-type layers may also be formed in situ by reaction of the neutral amine species with interlayer hydrogen or hydronium cations of the layered starting material. Alternatively, where the interlayer cations of the layered starting material are alkali metal cations, the organic ammonium cation may be formed by initially combining an amine and an aqueous acid solution, such as hydrochloric acid, and then treating the layered material with the resulting aqueous organoammonium ion solution. In either case, the treatment can be conducted in aqueous media so that water is then available to hydrolyze the electrically neutral, hydrolyzable polymeric oxide precursor subsequently introduced into the "propped" product.

Interspathic oxide pillars are then formed between the layers of the propped or swollen perovskite-type starting material and may include an oxide, preferably a polymeric oxide, of zirconium or titanium or more preferably of an element selected from Group IVB of the Periodic Table (Fischer Scientific Company Cat. No. 5-702-10, 1978), other than carbon, i.e., silicon, germanium, tin and lead. Other suitable oxides may include those of Group VA, e.g., V, Nb, and Ta, those of Group IIA, e.g., Mg or those of Group IIIB, e.g., B. Most preferably, the pillars include polymeric silica. In addition, the oxide pillars may include an element which provides catalytically active acid sites in the pillars, preferably aluminum.

The oxide pillars are formed from a precursor material which is preferably introduced between the layers of the organic "propped" species at 70 – 100°C, more preferably 80 – 90°C, and as a cationic, or more preferably, electrically neutral, hydrolyzable compound of the desired elements, e.g., those of Group IVB. The precursor material is preferably an organometallic compound which is a liquid under ambient conditions. In particular, hydrolyzable compounds, e.g., alkoxides, of the desired elements of the pillars are utilized as the

precursors. Suitable polymeric silica precursor materials include tetraalkylsilicates, e.g., tetrapropylorthosilicate, tetramethylorthosilicate and, most preferably, tetraethylorthosilicate. Where the pillars are also required to include alumina, a hydrolyzable aluminum compound can be contacted with the organic "propped" species before, after or simultaneously with the contacting of the propped layered oxide with the silicon compound. Preferably, the hydrolyzable aluminum compound employed is an aluminum alkoxide, e.g., aluminum isopropoxide. If the pillars are to include titania, a hydrolyzable titanium compound such as titanium alkoxide, e.g., titanium isopropoxide, may be used. In addition, the polymeric oxide precursor may contain zeolite precursors such that exposure to conversion conditions results in the formation of interspathic zeolite material as at least part of the oxide pillars.

After hydrolysis to produce the oxide pillars and calcination to remove the organic propping agent, the final pillared product may contain residual exchangeable cations. Such residual cations in the layered material can be ion exchanged by known methods with other cationic species to provide or alter the catalytic activity of the pillared product. Suitable replacement cations include cesium, cerium, cobalt, nickel, copper, zinc, manganese, platinum, lanthanum, aluminum, ammonium, hydronium and mixtures thereof.

When used as a catalyst, it may be desirable to incorporate the pillared product of the invention with another material, i.e. a matrix, resistant to the temperatures and other conditions employed in organic conversion processes. Such materials include active and inactive materials and synthetic or naturally occurring zeolites as well as inorganic materials such as clays, silica and/or metal oxide. Use of a matrix in conjunction with the pillared product, i.e. combined therewith, which is active, tends to improve the conversion and/or selectivity of the catalyst in certain organic conversion processes. Inactive materials suitably serve as diluents to control the amount of conversion in a given process so that products can be obtained economically without employing other means for controlling the rate of reaction. These materials may be incorporated into naturally occurring clays, e.g. bentonite and kaolin, to improve crush strength of the catalyst under commercial operating conditions. Said materials, i.e. clays, oxides, etc., function as binders for the catalyst. It is desirable to provide a catalyst having good crush strength because in commercial use it is desirable to prevent the catalyst from breaking down into powder-like materials. These clay binders have been employed normally only for the purpose of improving the crush strength of the catalyst.

Naturally occurring clays which can be composited with the pillared product include montmorillonite and kaolin families which in include the subbentonites, and the kaolins commonly known as Dixie, McNamee, Georgia and Florida clays or others in which the main mineral constitutent is halloysite, kaolinite, dickite, nacrite, or anauxite. Such clays can be used in the raw state as originally mined or initially subjected to calcination, acid treatment or chemical modification. Matrix materials useful for compositing with the pillared product also include inorganic oxides, notably alumina or silica.

In addition to the foregoing materials, the pillared product of the invention can be composited with a porous matrix material such as aluminum phosphate, silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia. The relative proportions of finely divided pillared product and inorganic oxide gel matrix vary widely, with the content of the pillared product ranging from 1 to 90 percent by weight and more usually, particularly when the composite is prepared in the form of beads or extrudates, in the range of 2 to 80 weight percent of the composite.

The present invention is illustrated further by the following Example.

**Example**

**Preparation of Perovskite-Related Layered Oxide $Ca_2Nb_3O_{10}$ Containing Interspathic Polymeric Silica**

$KCa_2Nb_3O_{10}$ was prepared by reacting a thoroughly ground mixture of 200 g $K_2CO_3$, 69.04 g $CaCO_3$ and 398.36 g $Nb_2O_5$ in a mole ratio of 1 : 4 : 3 at 750°C in air for 6 hours followed by 24 hours of heating at 1149°C. The material was cooled, reground and refired at 1149°C for 24 hours. 100 g of $KCa_2Nb_3O_{10}$ were then stirred in 300 ml of 6 MHCl for 24 hours at 60°C. The resulting solid was cooled, filtered, washed with water and dried overnight resulting in hydrated $HCa_2Nb_3O_{10}$. 30 grams of this material were stirred in 200 ml of water for 1 hour and 37.25 grams of n-octylamine were then added from a dropping funnel. The resulting mixture was heated to reflux and stirred for 24 hours. The reaction mixture was then filtered, washed with 1500 ml of hot water and dried in air overnight. An x-ray diffraction pattern of the powder from this reaction indicated a layer (d) spacing of 3.15 nm. The solid was then stirred in tetraethylorthosilicate (5 g TEOS g/solid) for 72 hours at 80°C. The material was filtered, air dried, and calcined for 4 hours at 500°C. An x-ray diffraction pattern of this powder exhibited a low angle d-spacing of 2.76 nm. The thickness of the $Ca_2Nb_3O_{10}$ layer was approximately 1.2 nm, leaving an interlayer opening of 1.56 nm.

**Claims**

1. A layered product comprising a layered oxide in which the layers have a perovskite-related

structure and pillars of an oxide of at least one element selected from Groups IB, IIB, IIIA, IIIB, IVA, IVB, VA, VB, VIA, VIIA and VIIIA of the Periodic Table of the Elements separating the layers of the layered oxide.

2. The product of claim 1 wherein the layered oxide has the formula:

$M_m[A_{n-1}B_nO_{3n+1}]$

where A is at least one metal atom capable of occupying 12-coordinate sites, B is a metal capable of occupying 6-coordinate sites and M is a counter-balancing cat ion of valence m, and where m is greater than zero and n is greater than or equal to 2.

3. The product of claim 2 wherein m is less than or equal to 1 and n is 2 – 7.

4. The product of claim 2 wherein A is a metal selected from Groups IA, IIA, IIIB and the lanthanides and B is a transition metal selected from Re and Groups IVB, VB and VIB.

5. The product of claim 1 wherein the layered oxide is $KCa_2Nb_3O_{10}$.

6. The product of claim 1 wherein the pillars comprise a polymeric oxide.

7. The product of claim 1 wherein the pillars comprise polymeric silica.

8. A method for preparing a layered product comprising a layered oxide in which the layers have a perovskite-related structure and pillars of an oxide of at least one element selected from Groups IB, IIB, IIIA, IVA, IVB, VA, VB, VIA , VIIA, and VIIIA of the Periodic Table separating the layers of the layered oxide which method comprises the steps of physically separating the layers of the layered oxide by introducing an organic cationic species between the layers at interlayer anionic sites associated with the layered oxide, introducing between the separated layers of the layered oxide a compound capable of conversion to an oxide and then converting said compound to the oxide to form oxide pillars separating adjacent layers of the layered oxide.

9. The method of claim 8 wherein said organic cationic species is an alkyl ammonium cation.

10. A catalyst composite comprising a layered product as claimed in claim 1 and a matrix material.

**Patentansprüche**

1. Schichtprodukt, das ein geschichtetes Oxid, worin die Schichten eine dem Perovskit verwandte Struktur aufweisen, und Stützen aus einem Oxid von zumindest einem Element umfaßt, das aus den Gruppen IB, IIB, IIIA, IIIB, IVA, IVB, VA, VB, VIA, VIIA und VIIIA des Periodensystems der Elemente ausgewählt ist, die die Schichten des geschichteten Oxids trennen.

2. Produkt nach Anspruch 1, worin das geschichtete Oxid die Formel aufweist:

$M_m[A_{n-1}B_nO_{3n+1}]$

worin A zumindest ein Metallatom ist, das die 12-Koordinatenplätze einnehmen kann, B ein Metall ist, das die 6-Koordinatenplätze einnehmen kann, und M ein Gegenkation der Wertigkeit m ist, und worin m größer als 0 und n größer als oder gleich 2 ist.

3. Produkt nach Anspruch 2, worin m kleiner als oder gleich 1 und n 2 bis 7 ist.

4. Produkt nach Anspruch 2, worin A ein Metall ist, das aus den Gruppen IA, IIA, IIIB und den Lanthaniden ausgewählt ist, und B ein Übergangsmetall ist, das aus Re und den Gruppen IVB, VB und VIB ausgewählt ist.

5. Produkt nach Anspruch 1, worin das geschichtete Oxid $KCa_2Nb_3O_{10}$ ist.

6. Produkt nach Anspruch 1, worin die Stützen ein polymeres Oxid umfassen.

7. Produkt nach Anspruch 1, worin die Stützen polymeres Siliziumdioxid umfassen.

8. Verfahren zur Herstellung eines Schichtprodukts, das ein geschichtetes Oxid, worin die Schichten eine dem Perovskit verwandte Struktur aufweisen, und Stützen eines Oxids von zumindest einem Element umfaßt, das aus den Gruppen IB, IIB, IIIA, IVA, IVB, VA, VB, VIA, VIIA und VIIIA des Periodensystems ausgewählt ist, die die Schichten des geschichteten Oxids trennen, wobei das Verfahren die Schritte umfaßt: physikalische Trennung der Schichten des geschichteten Oxids durch Einführung einer organischen kationischen Art zwischen die Schichten an den anionischen Zwischenschichtplätzen, die mit dem geschichteten Oxid verbunden sind, Einführung einer Verbindung zwischen die getrennten Schichten des geschichteten Oxids, die zur Umwandlung in ein Oxid in der Lage ist, und anschließende Umwandlung dieser Verbindung in das Oxid, um die Oxidstützen zu bilden, die die benachbarten Schichten des geschichteten Oxids trennen.

9. Verfahren nach Anspruch 8, worin die organische kationische Art ein Alkylammoniumkation ist.

10. Katalysatorverbundstoff, der ein geschichtetes Produkt nach Anspruch 1 und ein Matrixmaterial umfaßt.

**Revendications**

1. Un produit stratifié comprenant un oxyde stratifié dans lequel les couches ont une structure de type perovskite, et des piliers d'un oxyde d'au moins un élément choisi dans les Groupes IB, IIB, IIIA, IIB, IVA, IVB, VA, VB, VIA, VIIA et VIIIA du Tableau Périodique des Eléments, séparant les couches de l'oxyde stratifié.

2. Le produit selon la revendication 1, dans lequel l'oxyde stratifié à la formule

$M_m[A_{n-1}B_nO_{3n+1}]$

dans laquelle A est constitué d'au moins un atome métallique capable d'occuper des sites à coordination égale à 12, B et un métal capable d'occuper des sites à coordination égal à 6 et m est un cation d'équilibrage de valence m, m étant supérieur à zéro et n étant supérieur ou égal à 2.

3. Le produit selon la revendication 2, dans lequel m est inférieur ou égal à 1 et n vaut 2 – 7.

4. Le produit selon la revendication 2, dans lequel A est un métal choisi dans les Groupes IA, IIA, IIIB et les lanthanides, et B est un métal de transition choisi parmi Re et les métaux des Groupes IVB, VB et VIB.

5. Le produit selon la revendication 1, dans lequel l'oxyde stratifié est $KCa_2Nb_3O_{10}$.

6. Le produit selon la revendication 1, dans lequel les piliers comprennent un oxyde polymère.

7. Le produit selon la revendication 1, dans lequel les piliers comprennent de la silice polymère.

8. Un procédé pour la préparation d'un produit stratifié comprenant un oxyde stratifié dans lequel les couches ont une structure de type perovskite et sont séparées les unes des autres par des piliers contenant un oxyde d'au moins un élément choisi parmi les groupes IB, IIB, IIIA, IIIB, IVA, IVB, VA, VB, VIA, VIIA et VIIIA du Tableau Périodique, lequel procédé comprend les étapes consistant à séparer physiquement les couches de l'oxyde stratifié par introduction d'une espèce cationique organique entre les couches, en des sites anioniques interlamellaires, associés à l'oxyde stratifié, à introduire entre les couches séparées de l'oxyde stratifié un composé capable d'être converti en un oxyde, puis à convertir ledit composé en l'oxyde pour former des piliers d'oxydes, qui séparent les unes des autres les couches voisines de l'oxyde stratifié.

9. Le procédé selon la revendication 8, dans lequel ladite espèce cationique organique est un cation alkylammonium.

10. Un composite catalytique comprenant un produit stratifié selon la revendication 1 et un matériau formant matrice.